# EUROPEAN PATENT APPLICATION

(11) **EP 4 624 593 A1**
(43) Date of publication of application: **01.10.2025**
(21) Application number: 23889084.2
(22) Date of filing: 06.11.2023
(51) Int. Cl.: C12Q 1/6886, G01N 33/574

(54) **BIOMARKER FOR PREDICTING IMMUNOTHERAPEUTIC RESPONSIVENESS BASED ON SPATIAL TRANSCRIPTOME ANALYSIS AND USES THEREOF**

(30) Priority: 07.11.2022 KR 20220147120
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: JOUNG, Je Gun, Seoul 04354 (KR); KANG, Hae Youn, Seongnam-si Gyeonggi-do 13496 (KR); AN, Hee Jung, Seoul 06098 (KR); HWANG, So Hyun, Seoul 05823 (KR); PARK, Hyun, Seongnam-si Gyeonggi-do 13599 (KR); CHOI, Min Chul, Yongin-si Gyeonggi-do 16874 (KR); JOO, Won Duk, Seoul 06597 (KR); JEONG, Ju Yeon, Yongin-si Gyeonggi-do 16953 (KR)
(74) Representative: Rösler Rasch van der Heide & Partner
(86) International application number: PCT/KR2023/017681
(87) International publication number: WO 2024/101823

(57) **Abstract**

The present invention relates to a biomarker for predicting immunotherapeutic responsiveness based on spatial transcriptome analysis and uses thereof and, in particular, to: a marker composition for predicting the responsiveness of cancer patients to immunotherapy; a composition for predicting the responsiveness of cancer patients to immunotherapy; a kit for predicting the responsiveness of cancer patients to immunotherapy, comprising the composition; a method for providing information for predicting the responsiveness of cancer patients to immunotherapy; and a method for providing information for predicting the survival prognosis of cancer patients. The biomarker for predicting immunotherapeutic responsiveness, according to the present invention, was discovered by applying spatial transcriptome technology and analyzing cell group-specific gene expression values according to location information of cells in tissue sections, and can more precisely and accurately predict the responsiveness of cancer patients to immunotherapy and the survival prognosis of patients, thus enabling suitable treatments for patient groups, which may result in improved therapeutic effects and a reduction in pain and costs for patients.

## Description

### Technical Field

The present disclosure relates to a biomarker for predicting immunotherapy responsiveness based on spatial transcriptomic analysis, and a use thereof.

### Background Art

Cancer remains one of the most intractable diseases yet to be conquered by modern medicine. In South Korea, it is the leading cause of death among single diseases and imposes a significant socioeconomic burden. The incidence and mortality rates of cancer continue to rise. Current treatments for cancer include surgery, radiation therapy, and chemotherapy; however, these approaches often cause severe side effects such as cytotoxicity, metastasis, and recurrence, highlighting the need for novel therapeutic strategies.

Recently, the emergence of immune checkpoint antibodies, which modulate the immune environment, has brought increased attention to immunotherapy. In particular, immune cell therapeutics have established themselves as the "fourth modality" of cancer treatment, alongside surgery, radiation, and chemotherapy. They are actively being developed and clinically studied for more than ten types of cancers, including brain tumors, malignant melanoma, gastric cancer, urothelial carcinoma, head and neck cancer, liver cancer, and ovarian cancer.

Furthermore, the importance of biomarkers for predicting therapeutic response has grown in tandem with efforts to enhance the efficacy of immune cell therapeutics. The development of biomarkers to determine the applicability of immune cell therapy is currently underway.

In order to determine the suitability of immune cell therapy in cancer patients, mRNA expression profiles are measured to assess treatment responsiveness. Commonly used immune cell therapeutics include T cell therapies, NK cell therapies, and immune checkpoint inhibitors (PD-1, PD-L1, CTLA-4, etc.). The responsiveness to these therapeutics is influenced by the state of the tumor microenvironment (TME), which refers to the various surrounding cells (fibroblasts, immune cells, endothelial cells, etc.) within the tumor and the extracellular microcomponents that serve as constituents thereof.

However, current biomarkers for immune cell therapy, which are selected based on mRNA expression analysis from pathological cancer tissue slides, merely reflect the average gene expression across all cells within a given tissue or section, without considering the specific cellular composition of the tumor microenvironment. As a result, these biomarkers lack precision in predicting immunotherapy responsiveness, which can lead to unexpected adverse effects from inappropriate treatment decisions.

Accordingly, there is a need to develop novel biomarkers that more accurately and precisely predict immunotherapy responsiveness by taking into account the characteristics of the tumor microenvironment in relation to immune cell therapies.

### Disclosure of Invention

### Technical Problem

Leading to the present disclosure, intensive and thorough research conducted by the present inventors succeeded in selecting biomarkers capable of predicting the responsiveness of immune cell therapeutics to ovarian cancer using spatial transcriptomic analysis based on the GeoMx Digital Spatial Profiling (DSP) system and resulted in the finding that the use of the markers can enhance therapeutic efficacy in cancer patients and enable personalized treatment.

Therefore, an aspect of the present disclosure is to provide a marker composition for predicting immunotherapy responsiveness in cancer patients, the composition including at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, or a protein encoded by the gene.

Another aspect of the present disclosure is to provide a composition for predicting immunotherapy responsiveness in cancer patients, the composition comprising an agent capable of measuring an mRNA expression level or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G.

Yet another aspect of the present disclosure is to provide a kit for predicting immunotherapy responsiveness in cancer patients, the kit comprising the composition of the present disclosure.

Still another aspect of the present disclosure is to provide an information providing method for predicting immunotherapy responsiveness in a cancer patient, the method comprising the steps of: (1) measuring an mRNA expression level or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, from a biological sample isolated from a cancer patient; and (2) predicting the responsiveness of the patient to immunotherapy based on the measured mRNA or protein expression level.

A further aspect of the present disclosure is to provide an information providing method for the prognosis of survival in a cancer patient, the method comprising the step of measuring an mRNA expression level or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, from a biological sample isolated from the cancer patient.

### Solution to Problem

To achieve the goals described above, the present disclosure provides a marker composition for predicting immunotherapy responsiveness in cancer patients, the composition comprising at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, or a protein encoded by the gene.

In an embodiment of the present disclosure, the cancer may be selected from the group consisting of ovarian cancer, cholangiocarcinoma, colon cancer, adenocarcinoma, rectal cancer, breast cancer, synovial sarcoma, chondrosarcoma, thyroid cancer, gastric cancer, thymic cancer, cervical cancer, glioma, brain cancer, melanoma, lung cancer, bladder cancer, prostate cancer, leukemia, renal cancer, liver cancer, colorectal cancer, pancreatic cancer, lymphoma, uterine cancer, oral cancer, bronchial cancer, nasopharyngeal cancer, laryngeal cancer, skin cancer, hematologic cancer, parathyroid cancer, and ureteral cancer.

In an embodiment of the present disclosure, the immunotherapy may be selected from the group consisting of NK cell therapy, T cell therapy, CAR-immune cell therapy, DC vaccines, anti-PD-L1, anti-PD-1, and anti-CTLA-4.

The present disclosure also provides a composition for predicting immunotherapy responsiveness in cancer patients, the composition comprising an agent for measuring the mRNA or protein expression level of at least one gene selected from the group consisting of NKG7, ULBP3, FPR2, MYC, CXCL10, NECTIN2, CD8A, HLA-DQA1, BMP2, INF-b, TNF-b, IL6, OX40-L, OX40, Tim3, HLA-C, and HLA-G.

In an embodiment of the present disclosure, the gene expression level may be measured in a region of interest within a tumor microenvironment in a lesion tissue derived from a cancer patient.

In an embodiment of the present disclosure, the region of interest may be a tumor region where PanCK is expressed, an immune region where CD45 is expressed, or a stromal region.

In an embodiment, the expression level of NKG7, ULBP3, FPR2, or MYC may be measured in the immune region where CD45 is expressed; the expression level of CXCL10 or NECTIN2 may be measured in the tumor region where PanCK is expressed; and the expression levels of CD8A, HLA-DQA1, BMP2, INF-β, TNF-β, IL6, OX40-L, OX40, Tim3, HLA-C, or HLA-G may be measured in the stromal region.

In an embodiment of the present disclosure, the agent for measuring the mRNA expression level may be a primer or probe that specifically binds to the gene.

In an embodiment, the agent for measuring the protein expression level may be an antibody that specifically binds to the protein encoded by the gene.

The cancer may be selected from the group consisting of ovarian cancer, cholangiocarcinoma, colon cancer, adenocarcinoma, rectal cancer, breast cancer, synovial sarcoma, chondrosarcoma, thyroid cancer, gastric cancer, thymic cancer, cervical cancer, glioma, brain cancer, melanoma, lung cancer, bladder cancer, prostate cancer, leukemia, renal cancer, liver cancer, colorectal cancer, pancreatic cancer, lymphoma, uterine cancer, oral cancer, bronchial cancer, nasopharyngeal cancer, laryngeal cancer, skin cancer, hematologic cancer, parathyroid cancer, and ureteral cancer.

The immunotherapy may be selected from the group consisting of NK cell therapy, T cell therapy, CAR-immune cell therapy, DC vaccines, anti-PD-L1, anti-PD-1, and anti-CTLA-4.

In addition, the present disclosure also provides a kit for predicting immunotherapy responsiveness in cancer patients, the kit comprising the composition of the present disclosure.

The present disclosure also provides an information providing method for predicting immunotherapy responsiveness in a cancer patient, the method comprising: (1) measuring an mRNA expression level or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, from a biological sample isolated from the cancer patient; and (2) predicting the responsiveness of the cancer patient to immunotherapy based on the measured expression level.

In an embodiment of the present disclosure, step (1) may involve measuring an expression level in a region of interest within a tumor microenvironment in the biological sample.

In an embodiment of the present disclosure, the region of interest may be a tumor region where PanCK is expressed, an immune region where CD45 is expressed, or a stromal region.

In an embodiment of the present disclosure, the expression level of NKG7, ULBP3, FPR2, or MYC may be measured in the immune region where CD45 is expressed; the expression level of CXCL10 or NECTIN2 may be measured in the tumor region where PanCK is expressed; and the expression level of CD8A, HLA-DQA1, BMP2, INF-b, TNF-b, IL6, OX40-L, OX40, Tim3, HLA-C, or HLA-G may be measured in the stromal region.

According to an embodiment of the preset disclosure, the step (2) of predicting the responsiveness of the cancer patient to immunotherapy may be carried out by determining that the cancer patient has a high responsiveness to immunotherapy if the expression level of NKG7 or ULBP3 is equal to or greater than a defined expression threshold or if the expression level of FPR2 or MYC is equal to or less than the defined threshold.

According to an embodiment of the present disclosure, the step (2) of predicting the responsiveness of the cancer patient to immunotherapy may be carried out by determining that the cancer patient has a high responsiveness to immunotherapy if the expression level of CXCL10 or NECTIN2 is a defined threshold or less.

According to an embodiment of the present disclosure, the step (2) of predicting the responsiveness of the cancer patient to immunotherapy may be carried out by determining that the cancer patient has a high responsiveness to immunotherapy If the expression level of CD8A, HLA-DQA1, BMP2, INF-β, TNF-β, IL6, OX40-L, OX40, Tim3, HLA-C, or HLA-G is equal to or greater than a defined expression threshold.

Furthermore, the present disclosure provides an information providing method for the survival prognosis of a cancer patient, the method comprising the step of measuring the mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G from a biological sample isolated from the cancer patient.

In an embodiment, the expression level measured in a region of interest within the tumor microenvironment of the biological sample may be an expression level measured in a tumor region where PanCK is expressed, an immune region where CD45 is expressed, or a stromal region.

According to an embodiment, the cancer patient may be determined to have a high progression-free survival (PFS) if, in the immune region where CD45 is expressed, the expression level of NKG7 or ULBP3 is equal to or greater than a defined threshold or the expression level of FPR2 or MYC is equal to or less than the defined threshold.

According to an embodiment, the cancer patient may be determined to have a high progression-free survival (PFS) if the expression level of CXCL10 or NECTIN2 is a defined threshold or less in the tumor region where PanCK is expressed.

According to an embodiment, the cancer patient may be determined to have a high progression-free survival (PFS) if the expression level of CD8A, HLA-DQA1, BMP2, INF-β, TNF-β, IL6, OX40-L, OX40, Tim3, HLA-C, or HLA-G is equal to or greater than a defined threshold In the stromal region.

### Advantageous Effects of Invention

The present disclosure relates to biomarkers that enable the prediction of therapeutic responsiveness to immune cell therapy and the determination of treatment eligibility, as well as the prognosis of survival in cancer patients, based on the composition of the tumor microenvironment using samples obtained from the patients. The biomarkers provided by the present disclosure were identified through spatial transcriptomic technology, which analyzes cell-type-specific gene expression in tissue sections according to the spatial location of cells. Accordingly, the present biomarkers allow more precise and accurate prediction of immunotherapy responsiveness and survival prognosis in cancer patients, thereby enabling optimal treatment selection, improving therapeutic outcomes, and reducing the patient's suffering and medical costs.

### Brief Description of Drawings

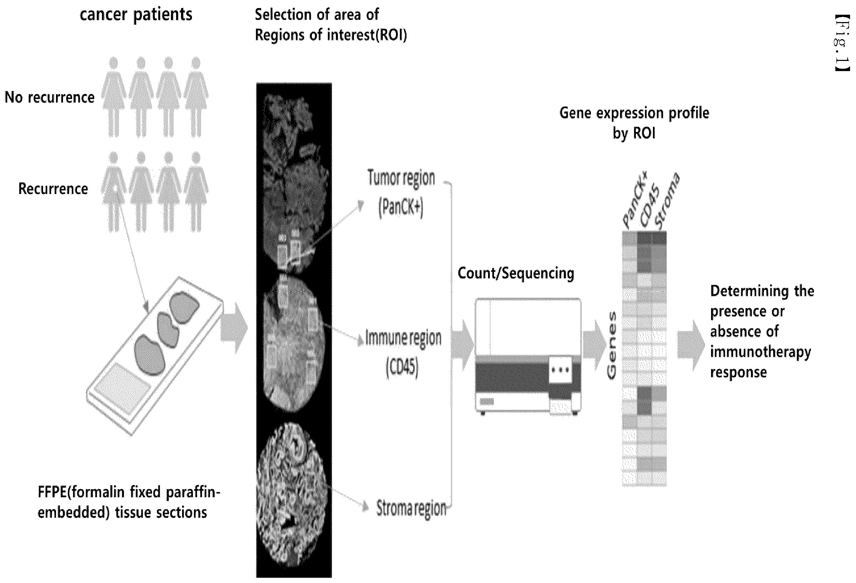

FIG. 1 is a schematic diagram illustrating the process of spatial transcriptomic analysis for selecting markers that exhibit differences in immunotherapy responsiveness in patients with solid tumors.

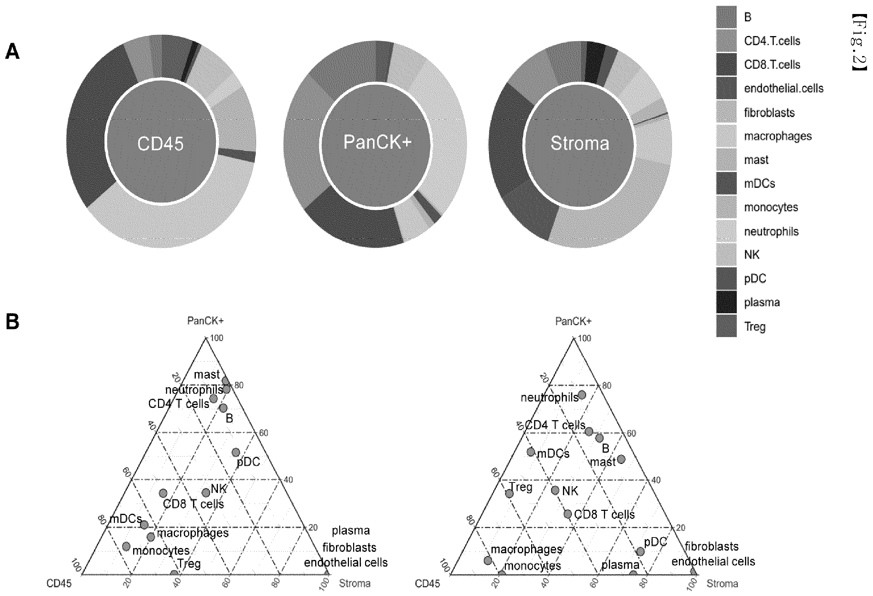

FIG. 2 shows the results of spatial transcriptomic analysis conducted on samples derived from patients with solid tumors. FIG. 2A is a pie chart representing the proportion of cell types in the regions of interest within the tumor microenvironment, namely the tumor region (PanCK), immune region (CD45), and stromal region. FIG. 2B is a triangular plot showing the frequency of cell types in each region of interest, wherein each dot represents a cell type.

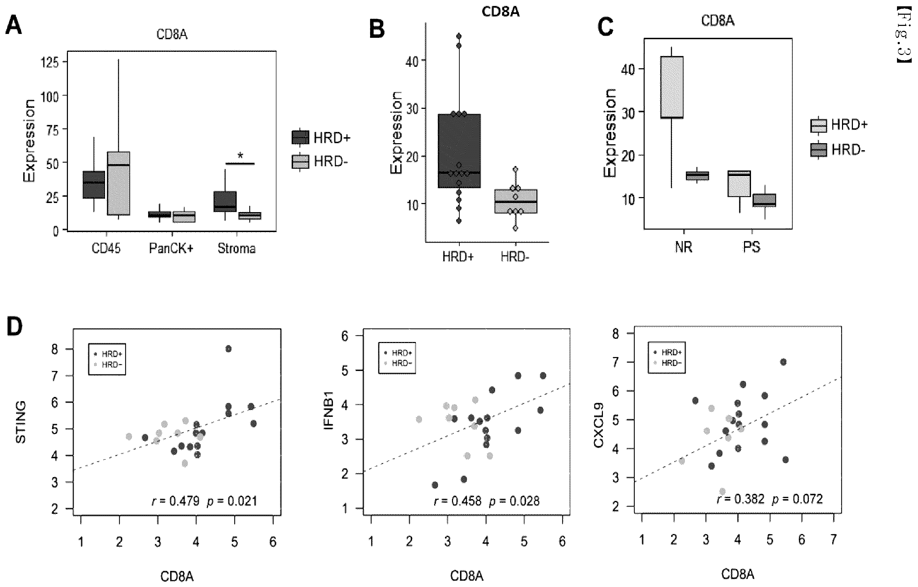

FIG. 3 shows the expression patterns of CD8A according to homologous recombination deficiency (HRD) status. FIG. 3A compares CD8A expression by HRD status across the three regions of interest. FIG. 3B compares CD8A expression in the stromal region by HRD status. FIG. 3C compares CD8A expression by HRD status in the NR (non-recurrent) and PS (platinum-sensitive) groups. FIG. 3D shows the correlation of CD8A with other markers (STING, IFNB1, and CXCL9). * p < 0.05.

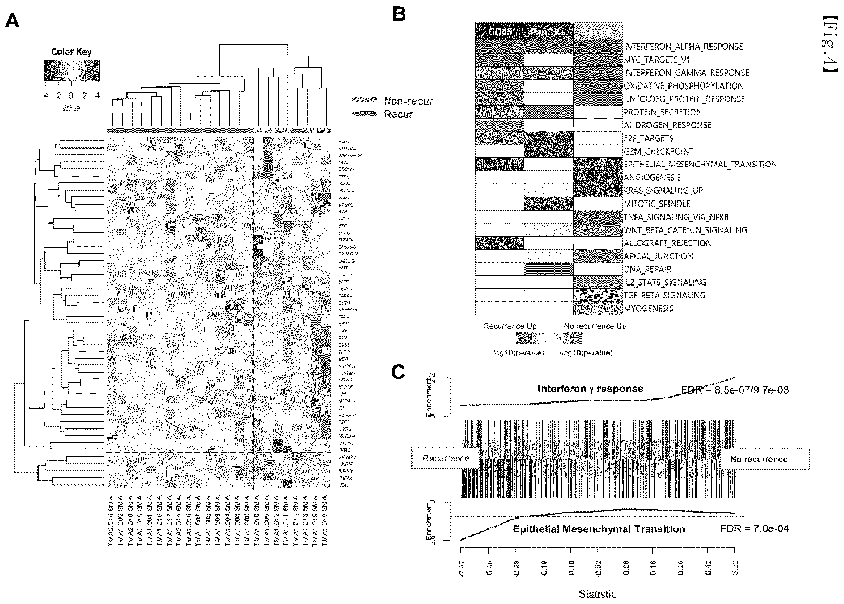

FIG. 4 shows transcriptomic differences between the non-recurrent group and the recurrent group. FIG. 4A is a heatmap of the top 50 differentially expressed genes in the stromal region. FIG. 4B shows enriched functions identified from characteristic gene sets in three different regions, where red indicates non-recurrent samples and blue indicates recurrent samples. FIG. 4C shows the results of gene set enrichment analysis for interferon-gamma response and epithelial-mesenchymal transition.

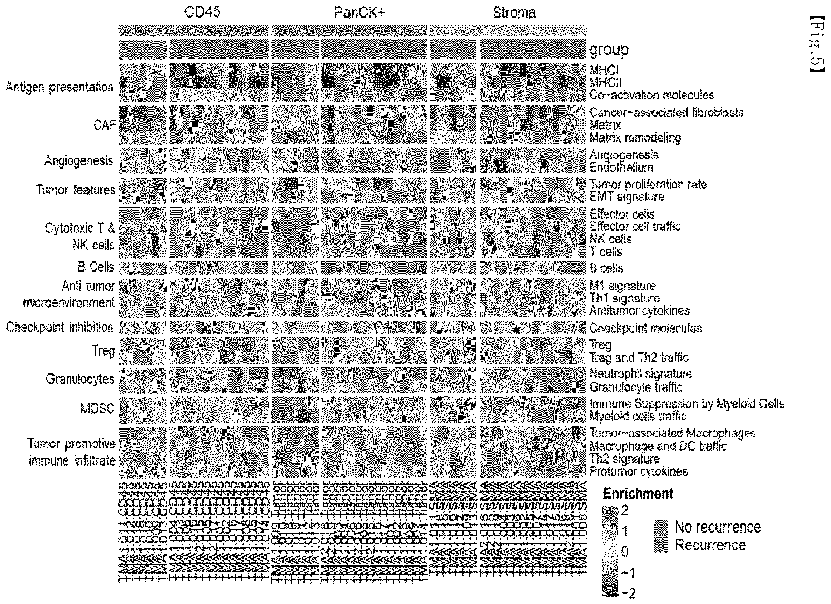

FIG. 5 shows the results of measuring gene expression signatures representing the functional composition of the tumor microenvironment between the non-recurrent and recurrent groups, categorized by regions of interest.

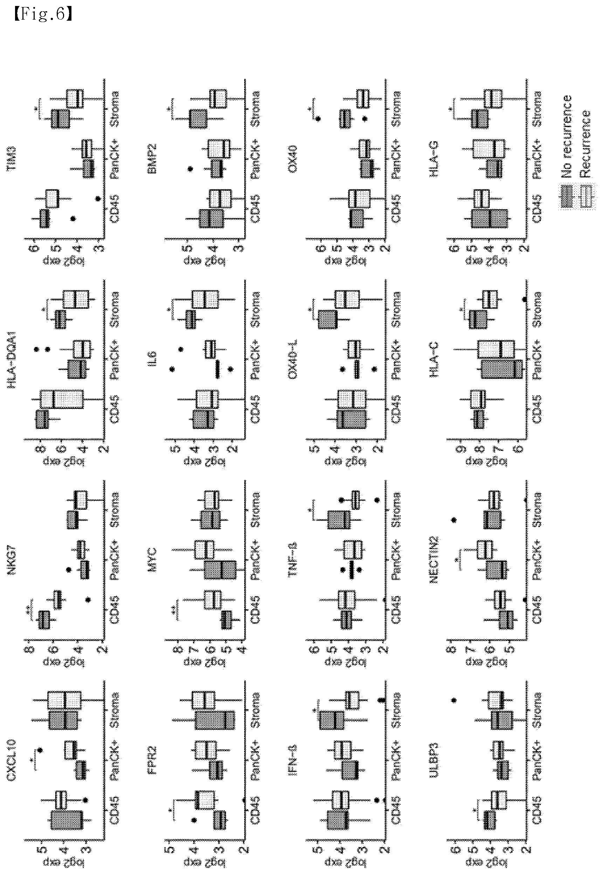

FIG. 6 shows the comparison of expression levels of selected genes related to immunotherapy responsiveness between the non-recurrent and recurrent groups.

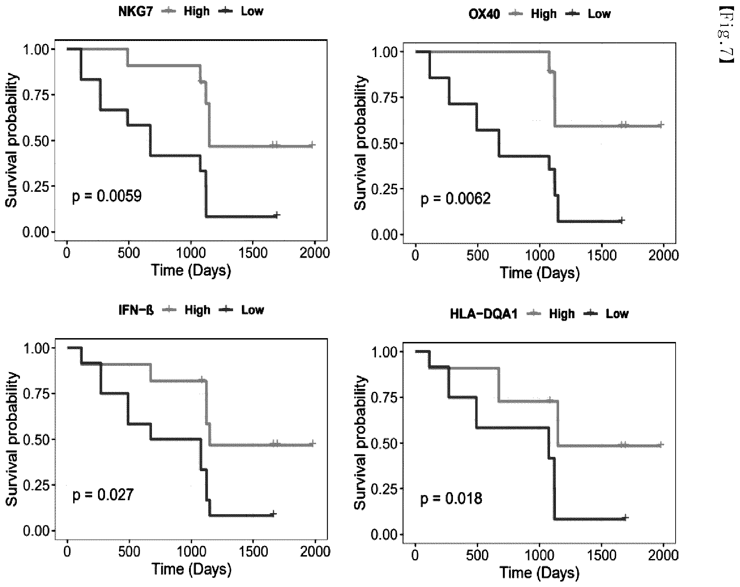

FIG. 7 shows the results of progression-free survival (PFS) analysis for the immunotherapy responsiveness prediction marker genes identified in the present disclosure, wherein the expression levels of NKG7, OX40, IFN-γ, and HLA-DQA1 were classified as low or high based on the median cutoff value.

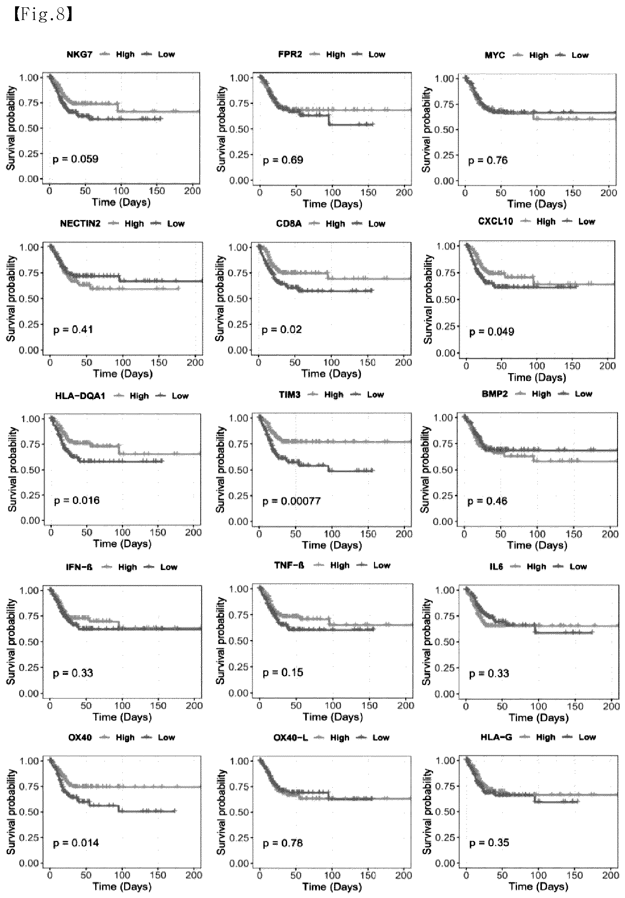

FIG. 8 shows the results of analyzing the association between the expression levels of immunotherapy responsiveness prediction marker genes identified in the present disclosure and patient survival prognosis using publicly available data from the TCGA (The Cancer Genome Atlas) cohort of cervical squamous cell carcinoma and endocervical adenocarcinoma.

### Best Mode for Carrying out the Invention

The present disclosure is characterized by providing novel biomarkers capable of predicting immunotherapy responsiveness in cancer patients.

Conventional biomarkers have primarily been identified by analyzing gene expression across all cells in cancer tissue slides, without taking into account the tumor microenvironment. As a result, such biomarkers tend to exhibit low diagnostic accuracy and may lead to unforeseen side effects after treatment decisions are made based on them.

In particular, it is often insufficient in the treatment of cancer to simply recognize and eliminate cancer-associated antigens. In many cases, the complex environment surrounding cancer cells, including surrounding cells (e.g., basal cells, inflammatory cells), blood vessels traversing the cells, and extracellular matrix, affects cancer cell survival and elimination. Therefore, the importance of cancer treatment technologies that consider the tumor microenvironment (TME) is increasing.

In light of this, research on methods to identify biomarkers capable of predicting immunotherapy responsiveness with consideration of the tumor microenvironment, conducted by the present inventors with the aim of enabling more precise and appropriate treatment for cancer patients resulted in discovering novel biomarkers, not identified by conventional methods, by performing spatial transcriptomic profiling analysis on pathological tissue and the tumor microenvironment using the GeoMx platform.

The term "tumor microenvironment" (TME), as used herein, refers to the cellular environment surrounding a tumor, including blood vessels and immune cells that are present around cancer cells and support the survival and growth of cancer cells, fibroblasts that provide structural support for cancer cells, bone marrow-derived inflammatory cells, various lymphocytes, signaling molecules, and components of the extracellular matrix, such as collagen and fibronectin.

According to an embodiment of the present disclosure, pathological tissues were obtained from patients who underwent standard treatment, including both recurrent and non-recurrent groups, to identify biomarkers capable of predicting immunotherapy responsiveness. Spatial transcriptomics technology was applied, unlike conventional approaches, to analyze gene expression patterns that differ between recurrent and non-recurrent groups following immunotherapy. Regions of interest within the tumor microenvironment were selected from tissue sections, cell type distributions in each region of interest were analyzed, and the expression levels of genes were profiled.

The term "region of interest" (ROI) refer to a specific area within the pathological tissue designated for analyzing cell-type distribution and transcriptomic patterns. In the present disclosure, the regions of interest (ROIs) were categorized into tumor regions, immune regions, and stromal regions. The tumor region refers to the area positive for PanCK expression, that is, a region in which PanCK is expressed; the immune region refers to an area positive for reaction with CD45 antibody; and the stromal region refers to an extracellular matrix area showing stromal positivity.

As a result of analyzing the distribution of cell types in each ROI with consideration of the tumor microenvironment, it was found that in the PanCK+ region, CD8 T cells accounted for 25.48% of all cells in the non-recurrent group and 16.40% in the recurrent group. In the stromal region, functions related to anti-tumor microenvironment, angiogenesis, and fibroblasts were found to be higher in the non-recurrent group than in the recurrent group.

Furthermore, transcriptomic analysis of genes showing expression differences between recurrent and non-recurrent groups in each ROI revealed that the 17 genes of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G exhibited statistically significant differences in expression.

These 17 genes were not identifiable through conventional bulk RNA sequencing analysis. Rather, they were discovered through precise transcriptomic analysis based on spatially defined regions of interest according to the present disclosure.

Accordingly, the present disclosure can provide a marker composition for predicting immunotherapy responsiveness in cancer patients, the composition comprising one or more genes selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, or proteins encoded by the genes.

Specifically, the names and NCBI GenBank accession numbers of the 17 biomarker genes identified for predicting immunotherapy responsiveness in cancer patients according to the present disclosure are as follows.

| Gene Symbol | Gene Name (full name) | NCBI GenBank No. (human) |
|---|---|---|
| NKG7 | Natural killer cell granule protein 7 | NM 005601 |
| ULBP3 | Homo sapiens ULI6 binding protein 3 | NM 024518 |
| FPR2 | N-formyl peptide receptor 2 | NM 001005738 |
| MYC | MYC proto-oncogene, bHLH transcription factor | NM_002467 |
| CXCL10 | C-X-C motif chemokine ligand 10 | NM 001565 |
| NECTIN2 | Poliovirus receptor-related 2 (PVRL2) | NM 001042724 |
| CD8A | Cluster of Differentiation 8a | NM 001145873 |
| HLA-DQA1 | Major histocompatibility complex, class II, DQ alpha 1 | NM_002122 |
| BMP2 | Bone Morphogenetic Protein 2 | NM 001200 |
| INF-b | Interferon beta 1 | NM.002176 |
| TNF-b | Tumor necrosis factor-beta | NM 000595 |
| IL6 | Interleukin 6 | NM 000600 |
| OX40-L | Tumor necrosis factor (ligand) superfamily, member 4 | NM_003326 |
| 0X40 | TNF receptor superfamily member 4 | NM 003327 |
| Tim3 | Hepatitis A virus cellular receptor 2 | NM 032782 |
| HLA-C | Major Histocompatibility Complex, Class I, C | NM 001243042 |
| HLA-G | Major Histocompatibility Complex, Class I, G | NM 001363567 |

Examples of the cancer diseases to which the biomarkers of the present disclosure can be applied include, but are not limited to, ovarian cancer, cholangiocarcinoma, colon cancer, adenocarcinoma, rectal cancer, breast cancer, synovial sarcoma, chondrosarcoma, thyroid cancer, gastric cancer, thymic cancer, cervical cancer, glioma, brain cancer, melanoma, lung cancer, bladder cancer, prostate cancer, leukemia, renal cancer, liver cancer, colorectal cancer, pancreatic cancer, lymphoma, uterine cancer, oral cancer, bronchial cancer, nasopharyngeal cancer, laryngeal cancer, skin cancer, hematologic cancer, parathyroid cancer, and ureteral cancer, with preference for ovarian cancer.

In the present disclosure, "predicting responsiveness to immunotherapy in a cancer patient" refers to predicting whether the patient will respond favorably or unfavorably to an immunotherapeutic agent (e.g., an immune checkpoint inhibitor), predicting the risk of resistance to the immunotherapeutic agent, and predicting the patient's prognosis after immunotherapy, such as recurrence, metastasis, and survival.

As used herein, the term "immunotherapeutic agent" refers to an anticancer agent that activates immune cells in the human body to destroy cancer cells, and may refer to an immune-oncology drug that exerts a therapeutic effect by enhancing the patient's own immune response.

The immunotherapeutic agent may be an immune cell therapeutic, which may be selected from the group consisting of NK cell therapies, T cell therapies, CAR-immune cell therapies, dendritic cell (DC) vaccines, anti-PD-L1, anti-PD-1, and anti-CTLA-4, but with no limitations thereto.

The present disclosure may also provide a composition for predicting immunotherapy responsiveness in a cancer patient, the composition comprising an agent capable of measuring the mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G.

The gene expression level may be measured in a region of interest within the tumor microenvironment in lesion tissue derived from a cancer patient, and the region of interest may be a tumor region where PanCK is expressed, an immune region where CD45 is expressed, or a stromal region, as described above.

According to an embodiment of the present disclosure, analysis of markers that exhibited differential gene expression between the recurrent and non-recurrent groups after immunotherapy in each region of interest revealed that NKG7, ULBP3, FPR2, and MYC were differentially expressed in the immune region; CXCL10 and NECTIN2 in the tumor region; and CD8A, HLA-DQA1, BMP2, INF-β, TNF-β, IL6, OX40-L, OX40, Tim3, HLA-C, and HLA-G in the stromal region.

As used herein, an "agent capable of measuring gene expression level" refers to an agent that directly or indirectly measures the expression of a gene or the protein encoded thereby in a patient sample to determine the degree of expression for the purpose of predicting responsiveness to immunotherapy (immune-oncology drug). The "gene expression level" may be determined by measuring the expression level of the gene's mRNA or the protein encoded by the gene. The gene expression level may serve as a biomarker, and more specifically, the therapeutic responsiveness to immunotherapy in a cancer patient may be determined based on the expression level of the relevant gene.

In addition, "measurement of mRNA expression level" refers to a process of identifying the presence and expression level of the mRNA of the gene in a sample from a subject, and may include quantifying the amount of mRNA. The agent capable of measuring the mRNA expression level may be a primer or probe that specifically binds to the gene.

Any method known in the art may be used to measure the mRNA expression level, such as reverse transcription polymerase chain reaction (RT-PCR), competitive RT-PCR, real-time RT-PCR, RNase protection assay (RPA), northern blotting, or DNA chip analysis. In an embodiment of the present disclosure, the GeoMx Digital Spatial Profiler (DSP) analysis method was used.

The "measurement of protein expression level" refers to a process of determining the presence and expression level of the protein encoded by the gene in a sample from a subject, and may include quantifying the amount of the protein. Any method known in the art may be used to measure the protein expression level, such as western blotting, enzyme-linked immunosorbent assay (ELISA), radioimmunoassay (RIA), radioimmunodiffusion, Ouchterlony double immunodiffusion, rocket immunoelectrophoresis, immunohistochemistry, immunoprecipitation assay, complement fixation assay, fluorescence-activated cell sorting (FACS), or protein chip analysis. The agent capable of measuring the protein expression level may be an antibody that specifically binds to the protein encoded by the gene.

The present disclosure may also provide a kit for predicting immunotherapy responsiveness in cancer patients, the kit comprising the composition of the present disclosure.

The kit may detect markers by determining the mRNA expression level or protein expression level of the gene. Additionally, the kit may include primers or probes for measuring the expression level of the gene that increases or decreases depending on the patient's responsiveness to immunotherapy, or optionally, antibodies that specifically recognize the marker, along with one or more other component compositions, solutions, or devices suitable for the analysis method.

For example, a kit for measuring the mRNA expression level of the gene may be a kit that includes essential components for performing RT-PCR. The RT-PCR kit may include, in addition to specific primers for the gene, a test tube or other suitable container, reaction buffer, deoxynucleotides (dNTPs), enzymes such as Taq polymerase and reverse transcriptase, DNase, RNase inhibitors, DEPC-treated water, and sterile water.

Additionally, a kit for measuring protein expression levels may include a substrate for immunological detection using an antibody, an appropriate buffer, a secondary antibody labeled with a chromogenic enzyme or fluorescent molecule, and a chromogenic substrate. The substrate may be a nitrocellulose membrane, a 96-well plate made of polyvinyl resin, a 96-well plate made of polystyrene resin, or a glass slide. The chromogenic enzyme may be peroxidase or alkaline phosphatase, the fluorescent molecule may be FITC or RITC, and the chromogenic substrate may be ABTS (2,2'-azino-bis(3-ethylbenzothiazoline-6-sulfonic acid)), OPD (o-phenylenediamine), or TMB (tetramethylbenzidine).

Furthermore, the present disclosure may provide an information providing method for predicting immunotherapy responsiveness in a cancer patient, the method comprising the step of measuring the expression level of a marker gene or the protein encoded thereby.

Specifically, the information providing method for predicting immunotherapy responsiveness in a cancer patient according to the present disclosure comprises the steps of: (1) measuring an mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G from a biological sample isolated from the cancer patient; and (2) predicting the responsiveness of the cancer patient to immunotherapy based on the measured mRNA or protein expression level.

In an embodiment of the present disclosure, tissue microarrays (TMAs) were constructed considering the tumor microenvironment using pathological tissue samples obtained from cancer patients. Marker genes that showed significant expression differences between the recurrent (non-responsive) and non-recurrent (responsive) groups were selected from each region of interest. In the immune region expressing CD45, NKG7 and ULBP3 showed higher expression in the non-recurrent group compared to the recurrent group, while FPR2 and MYC showed lower expression.

In the tumor region expressing PanCK, CXCL10 and NECTIN2 showed lower expression in the non-recurrent group compared to the recurrent group.

In the stromal region, which is another region of interest, CD8A, HLA-DQA1, BMP2, INF-β, TNF-β, IL6, OX40-L, OX40, Tim3, HLA-C, and HLA-G showed higher expression in the non-recurrent group compared to the recurrent group.

Based on these results, the inventors confirmed that immunotherapy responsiveness in cancer patients can be predicted by analyzing the expression level or pattern of these genes in specific regions of interest, which can also guide the decision to administer immunotherapeutic agents.

Accordingly, if the expression level of NKG7 or ULBP3 in the immune region is equal to or greater than a defined expression threshold or if the expression level of FPR2 or MYC is equal to or less than the defined threshold, the cancer patient is determined to have high responsiveness to immunotherapy, and administration of an immunotherapeutic agent may be decided.

Likewise, if the expression level of CXCL10 or NECTIN2 in the tumor region is equal to or less than a defined expression threshold, the patient may be determined to have high responsiveness to immunotherapy and receive immunotherapeutic treatment.

Furthermore, if the expression level of CD8A, HLA-DQA1, BMP2, INF-β, TNF-β, IL6, OX40-L, OX40, Tim3, HLA-C, or HLA-G in the stromal region is equal to or greater than a defined expression threshold, the patient may be determined to be highly responsive to immunotherapy, and treatment may be initiated accordingly.

As used herein, the term "defined expression threshold" refers to the optimal threshold expression value of a specific marker gene that distinguishes between the recurrent and non-recurrent groups. This threshold is determined by logistic regression to maximize both sensitivity and specificity. Preferably, the threshold is determined by analyzing a sufficiently large sample size to account for statistical variability in the predictive model.

In the present invention, immunotherapy responsiveness in a cancer patient can be predicted by comparing the expression level of the marker gene against the defined expression threshold.

In an embodiment, the defined expression threshold values for the 17 marker genes are shown in Table 3.

For instance, if the expression value of the NKG7 gene in the immune region is equal to or greater than 6.138 (the defined threshold value), the patient is determined to have high responsiveness to the immunotherapeutic agent and treatment may be initiated.

As another example, if the expression value of the CXCL10 gene in the tumor region is equal to or less than 3.478 (the defined threshold value), the patient is determined to be highly responsive to immunotherapy and may be treated accordingly.

Furthermore, the present disclosure may provide an information providing method for the survival prognosis of cancer patients, the method comprising measuring an mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G from a biological sample isolated from the cancer patient.

By analyzing the expression level of the gene in a specific region of interest, not only can immunotherapy responsiveness be predicted, but also the survival prognosis of the cancer patient.

According to the survival prognosis of a cancer patient of the present disclosure, if the expression level of NKG7 or ULBP3 in the immune region expressing CD45 is equal to or greater than the defined threshold, or the expression level of FPR2 or MYC is equal to or less than the defined threshold, the cancer patient may be determined to have a high progression-free survival (PFS).

Additionally, if the expression level of CXCL10 or NECTIN2 in the PanCK-positive tumor region is equal to or less than the defined threshold, the cancer patient may also be determined to have high PFS.

Furthermore, if the expression level of CD8A, HLA-DQA1, BMP2, INF-b, TNF-b, IL6, OX40-L, OX40, Tim3, HLA-C, or HLA-G in the stromal region is equal to or greater than the defined threshold, the patient may be determined to have high PFS.

In an embodiment of the present disclosure, the association between the expression of the selected marker genes and cancer patient survival was analyzed. Consistent with the immunotherapy responsiveness results, higher expression of NKG7 in the CD45-positive immune region was associated with higher PFS, indicating a favorable survival prognosis. In another embodiment, higher expression of OX40, INF-β, and HLA-DQA1 in the stromal region also correlated with higher PFS, indicating a favorable prognosis.

These results demonstrate that the biomarkers identified through spatial transcriptomic analysis according to the present disclosure were discovered with consideration of the tumor microenvironment, and thus allow for more accurate and precise prediction of both immunotherapy responsiveness and survival prognosis in cancer patients.

### Mode for Carrying out the Invention

Below, a better understanding of the present disclosure may be obtained through the following Examples, which are set forth to illustrate, but are not to be construed to limit, the scope of the present disclosure.

### <Experimental Methods>

### Patients and Tissue Microarray (TMA) construction

The experiments of the present disclosure included 15 patients with ovarian cancer who were treated with standard therapy. Response to standard therapy was evaluated and classified as no recurrence (NR, n=4), platinum sensitive (PS, n=8), platinum resistance (PR, n=1), and refractory (RF, n=2). For Tissue microarray (TMA) construction, hematoxylin and eosin staining was conducted to select representative tumor areas and tumors were reviewed by a pathologist. Tumor core selection was not based on specific tumor segments or location. Table 1 summarizes the clinicopathologic characteristics of the patients included in the experiment.

**TABLE 1**

| **Variables** | **Number (%) or median (range)** |
|---|---|
| **Stage** | |
| II | 1 (6.7) |
| III | 12 (80.0) |
| IV | 2 (13.3) |

| **HRD status** | |
|---|---|
| Wild | 5 (33.3) |
| BRCA1/2 mutation | 8 (53.3) |
| Other mutations | 2 (13.3) |

| **Response** | |
|---|---|
| NR | 4 (26.7) |
| PS | 1 (6.7) |
| PR | 8 (53.3) |
| RF | 2 (13.3) |

| | |
|---|---|
| NR, No recur; PS, Platinum-sensitive; PR, Platinum-resistant; RF, Refractory | |

### Digital spatial profiling (DSP)

The tissue microarrays (TMAs) obtained from gynecologic cancer patients were analyzed using Human Whole Transcriptome Atlas of NanoString GeoMx profiling Reagents (NanoString Technologies Inc., Seattle, WA, USA). RNA probes with target-specific sequences were covalently attached to the TMA (in situ hybridization (ISH)), and then incubated with specific morphology markers conjugated to photocleavable (PC)-oligos cocktail. In this Example, the following specific morphology markers were used for TMA profiling: TMA 1: PanCK, SMA, PTPRC (CD45), DNA, and TMA 2: KRT5, ACTA2, PTPRC (CD45), DNA. The labeled TMAs were scanned on a GeoMx Digital Spatial Profiling (DSP) system (NanoString Technologies, USA) to confirm overall tissue architecture.

Regions of interest (ROIs) were designated or were automatically assigned for multiplex profiling using fluorescence labeling. Following automated tissue segmentation, oligonucleotides from selected ROIs were released via localized UV light exposure. The photocleaved oligos were collected via microcapillary aspiration and dispensed into 96-well plates. The localized UV exposure and photocleaved oligo collection were repeated for each area of interest. The oligos collected from each area of illumination (AOI) were amplified using PCR, and the PCR products were purified using AMPure XP beads (Beckman Coulter Diagnostics Inc., CA, USA). After pooling the purified libraries, sequencing was performed.

Library quantity & quality were assessed using a Qubit 4.0 fluorometer (Thermo Fisher Scientific, Waltham, MA, USA) and a TapeStation 4200 system (Agilent Technologies, Santa Clara, CA, USA). Sequencing was performed on the NovaSeq 6000 platform (Illumina, San Diego, CA, USA).

### Data processing

Sequencing data from the digital spatial profiling analysis were processed using the GeoMx NGS pipeline. Reads were aligned and matched to unique IDs of the probe. PCR duplicates were removed using unique molecular identifiers, and the remaining reads were converted into digital counts. The presence or absence of genes was defined by the limit of quantitation (LOQ), calculated as the geometric mean of negative probes multiplied by the square of the geometric standard deviation. Among the 18,677 genes analyzed, 16,132 genes (86.4%) exceeded the LOQ in at least 10% of AOIs. Q3 normalization (75th percentile of counts per sample) was used for downstream analyses to account for AOI area variation.

### Gene expression analysis

To analyze cell abundance in spatial gene expression datasets from each ROI, a constrained log-normal regression-based deconvolution was performed using the SpatialDecon R package (Griswold, 2022). The SafeTME matrix, which contains cell profiles of immune and stromal cell types commonly found in tumors, was used for deconvolution (Danaher, 2022). Differentially expressed genes were identified using the edgeR R package (Robinson, 2010), and gene set differences between response groups were determined using the camera function in the limma R package (Ritchie, 2015). The Hallmark gene set collection from MSigDB (Liberzon, 2015) was used.

A pie chart for data visualization was generated using ggplot2 R package. A ternary plot was created using the ggtern package in order to present cell type abundance for AOI (Hamilton, 2018). The relative expression levels of functional gene expression signatures (Fges) in the tumor microenvironment was analyzed using the single-sample gene set enrichment analysis (ssGSEA) algorithm for each sample (Bagaev, 2021).

### Statistical tests

The survival analysis was displayed using the Cox proportional hazards model in the survival R package. Statistical comparisons between two groups were performed using the Wilcoxon rank-sum test.

### <EXAMPLE 1>

### Gene profile generation by region of interest through spatial transcriptome analysis

To select gene markers (immunotherapy response markers) associated with immunogenetic responses in ovarian cancer patients, formalin-fixed paraffin-embedded (FFPE) slides were prepared from lesion tissues of 15 ovarian cancer patients who received standard therapy. After constructing tissue microarrays (TMAs), tumor, immune, and stromal regions were distinguished and visualized using respective fluorescent morphology markers for PanCK+, CD45, and Stroma with the NanoString GeoMx Digital Spatial Profiler. Then, a total of 75 regions of interest (ROIs) were selected, each 1 to 2 per segment per patient, and RNA sequencing was performed. Table 2 shows the analysis results of the proportion of cell types per segment for PanCK+, CD45, and Stroma.

**TABLE 2**

| type | Total | | | No recurrence | | | Recurrence | | |
|---|---|---|---|---|---|---|---|---|---|
| | CD45 | PanCK+ | Stroma | CD45 | PanCK+ | Stroma | CD45 | PanCK+ | Stroma |
| macrophages | 35.910 | 4.710 | 7.270 | 39.350 | 9.680 | 12.190 | 34.300 | 2.540 | 5.110 |
| mast | 0.090 | 1.190 | 0.740 | 0.020 | 1.620 | 0.340 | 0.130 | 1.000 | 0.920 |
| B | 2.130 | 13.070 | 6.290 | 1.310 | 11.830 | 3.690 | 2.510 | 13.610 | 7.430 |
| plasma | 1.090 | 0.000 | 3.420 | 0.000 | 0.000 | 0.710 | 1.600 | 0.000 | 4.610 |
| CD4.T.cells | 4.470 | 22.750 | 8.400 | 2.710 | 20.680 | 4.460 | 5.290 | 23.660 | 10.130 |
| CD8.T.cells | 29.100 | 19.170 | 18.970 | 37.250 | 25.480 | 11.650 | 25.290 | 16.400 | 22.180 |
| NK | 6.750 | 6.340 | 4.830 | 5.290 | 5.580 | 5.320 | 7.430 | 6.680 | 4.620 |
| pDC | 0.580 | 0.720 | 2.230 | 0.360 | 1.530 | 1.080 | 0.680 | 0.370 | 2.730 |
| mDCs | 1.650 | 1.670 | 0.290 | 1.440 | 0.470 | 0.330 | 1.750 | 2.190 | 0.280 |
| monocytes | 10.270 | 0.370 | 2.460 | 7.920 | 1.230 | 1.240 | 11.370 | 0.000 | 2.990 |
| neutrophils | 2.520 | 27.240 | 5.800 | 0.710 | 21.900 | 5.400 | 3.370 | 29.570 | 5.970 |
| Treg | 5.350 | 2.510 | 1.130 | 3.510 | 0.000 | 2.090 | 6.210 | 3.610 | 0.710 |
| endothelial.cells | 0.000 | 0.000 | 10.540 | 0.000 | 0.000 | 17.250 | 0.000 | 0.000 | 7.600 |
| fibroblasts | 0.100 | 0.250 | 27.630 | 0.140 | 0.000 | 34.250 | 0.080 | 0.370 | 24.730 |

As a result of the analysis, the proportions of cell types identified in the captured regions of ovarian cancer were analyzed using deconvolution methods. As shown in FIG. 2A and Table 2, CD8 T cells and macrophages were abundantly distributed in the CD45 region (35.91% and 29.10%, respectively), while in the PanCK+ region, CD4 T cells, CD8 T cells, and neutrophils were relatively abundant (22.75%, 19.17%, and 27.24%, respectively). In the stromal region, fibroblasts, endothelial cells, and CD8 T cells were relatively enriched (27.63%, 10.54%, and 18.97%, respectively).

Additionally, analysis of the distribution of each cell type between non-recurrent (responder) and recurrent (non-responder) groups in each region (PanCK+, CD45, and Stroma) revealed that, as shown in FIG. 2B, the proportion of CD8 T cells in the PanCK+ region was 25.48% in the non-recurrent group, compared to 16.40% in the recurrent group.

### <EXAMPLE 2>

### Identification of immunotherapeutic markers regarding spatial information

Analysis was made of markers associated with the tumor microenvironment under homologous recombination-deficiency (HRD) conditions.

As a result, CD8A expression showed a significant difference in the stromal compartment depending on HRD status (P=0.019) (FIGS. 3A and 3B), while no significant difference was observed in other compartments. This distinction was more pronounced in the non-recurrent group than in the platinum-sensitive group (FIG. 3C). CD8A expression was also found to be highly correlated with STING pathway-related genes (STING, IFNB1, and CXCL9) (FIG. 3D).

Furthermore, differentially expressed genes between responder groups in each compartment were identified (FIG. 4A), and gene function distributions from characteristic gene sets in three compartments were confirmed (FIG. 4B). Gene sets "related to interferon-alpha/gamma response" were found to be widely distributed in all compartments. The gene sets of the interferon-alpha/gamma response were activated in the non-recurrent group, whereas "epithelial-mesenchymal transition (EMT)" was activated in the recurrent group (FIG. 4C).

In addition, the functional components of the tumor microenvironment including anti-tumor microenvironment, angiogenesis and the fibroblast are shown strongly in responder group (non-recurrent) rather than non-responder (recurrent) (FIG. 5).

### <EXAMPLE 3>

### Selection of Immunotherapy Response Markers with Spatial Information by Region of Interest

For immune response-related genes, biomarker candidates were screened in each region of interest (CD45, PanCK, and Stroma). Specifically, excluding normal tissue, genes showing statistically significant expression differences between non-recurrent and recurrent groups in each ROI were selected using the Wilcoxon rank sum test. The results are shown in Table 3.

**TABLE 3**

| ROI | Marker | Expression Ratio* | *P*-value | Threshold | Function |
|---|---|---|---|---|---|
| CD45 | NKG7 | High | 0.0011 | 6.138 | IFN-γ-responsive |
| CD45 | FPR2 | Low | 0.048 | 3.806 | M1 macrophage |
| CD45 | MYC | Low | 0.0074 | 5.455 | Suppressed T cell infiltration |
| CD45 | ULBP3 | High | 0.034 | 3.599 | NKR ligand |
| PanCK+ | CXCL10 | Low | 0.018 | 3.478 | Chemokine ligands |
| PanCK+ | NECTIN2 | Low | 0.039 | 5.608 | NKR ligand |
| Stroma | CD8A | High | 0.012 | 4.116 | Cytotoxic activity, Th1 signaling |
| Stroma | HLA-DQA1 | High | 0.025 | 4.723 | Antigen presentation |
| Stroma | BMP2 | High | 0.012 | 4.368 | bone morphogenic protein (BMP) family |
| Stroma | INF-b | High | 0.023 | 4.705 | Anti-tumor (Proinflammation) |
| Stroma | TNF-b | High | 0.041 | 4.806 | Anti-tumor (Proinflammation) |
| Stroma | IL6 | High | 0.032 | 3.551 | Pro-tumor (Immune suppression) |
| Stroma | OX40-L | High | 0.04 | 3.551 | T-cell co-stimulator |
| Stroma | OX40 | High | 0.012 | 3.728 | T-cell co-stimulator |
| Stroma | Tim3 | High | 0.038 | 4.766 | Immune Check Point |
| Stroma | HLA-C | High | 0.049 | 8.148 | NKR ligand |
| Stroma | HLA-G | High | 0.045 | 3.777 | NKR ligand |

| | | | | | |
|---|---|---|---|---|---|
| * fold change (no-recurrence/recurrence), ** log2(expression) | | | | | |

In Table 3, the "Expression Ratio" column indicates whether the gene expression level was higher or lower in the non-recurrent group compared to the recurrent group. The "P-value" column provides the defined expression threshold that optimally separates the two groups, determined via logistic regression to maximize sensitivity and specificity. The "Function" column indicates the role of each marker in the tumor microenvironment.

As shown in Table 3 and FIG. 6, four genes (NKG7, ULBP3, FPR2, and MYC) showed expression differences between groups in the CD45 region, while CXCL10 and NECTIN2 were lower in the PanCK+ region in the non-recurrent group. Eleven genes (CD8A, HLA-DQA1, BMP2, INF-β, TNF-β, IL6, OX40-L, OX40, Tim3, HLA-C, and HLA-G) were more highly expressed in the stromal region in the non-recurrent group compared to the recurrent group.

The 17 markers selected on the basis of the data obtained above were found to show statistically significant expression differences between the recurrent and non-recurrent groups in specific ROIs of tissue samples from ovarian cancer patients. These biomarkers were not identifiable through conventional RNA-seq-based gene expression analyses, demonstrating their potential use in predicting immunotherapy responsiveness in solid tumors, particularly ovarian cancer.

### <EXAMPLE 4>

### Survival Validation Using Selected Immunotherapy Response Markers

Moreover, survival analysis was conducted using the immunotherapy response markers identified in Example 3 to evaluate their potential for predicting clinical prognosis.

As shown in FIG. 7, progression-free survival (PFS) was analyzed based on the expression levels of NKG7, OX40, INF-β, and HLA-DQA1. NKG7 showed a favorable prognosis in the group with high expression (greater than the median) in the CD45 region (P=0.0059). Similarly, OX40, INF-β, and HLA-DQA1 showed favorable prognostic values in the stromal region with P-values of 0.0062, 0.027, and 0.018, respectively.

The relevance between the markers selected in the present disclosure and the survival prognosis was verified using public data from the Cervical Squamous Cell Carcinoma and Endocervical Adenocarcinoma cohort of The Cancer Genome Atlas (TCGA) large-scale oncogenomic database (FIG. 8).

Consistent with previous results, high expression levels of NKG7, OX40, INF-β, and HLA-DQA1 were associated with favorable prognosis, with significance of P= 0.059, 0.014, 0.33, and 0.016, respectively.

These findings indicate that the markers capable of predicting the responsiveness of cancer patients to immune cell therapeutics in the present disclosure were identified based on the spatial composition of the tumor microenvironment and may be useful for accurately and precisely predicting both immunotherapy responsiveness and survival prognosis in cancer patients.

The preferred embodiments of the present disclosure have been described above. However, those skilled in the art will understand that various modifications can be made to the invention without departing from the essential spirit thereof. Therefore, the disclosed embodiments should be understood as illustrative rather than limiting. The scope of the invention is defined by the claims, and all equivalents thereof should be interpreted as falling within the scope of the present disclosure.

## Claims

1. A marker composition for predicting immunotherapy responsiveness in a cancer patient, comprising at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G, or proteins encoded by the genes.

2. The marker composition of claim 1, wherein the cancer is selected from the group consisting of ovarian cancer, cholangiocarcinoma, colon cancer, adenocarcinoma, rectal cancer, breast cancer, synovial sarcoma, chondrosarcoma, thyroid cancer, gastric cancer, thymic cancer, cervical cancer, glioma, brain cancer, melanoma, lung cancer, bladder cancer, prostate cancer, leukemia, renal cancer, liver cancer, colorectal cancer, pancreatic cancer, lymphoma, uterine cancer, oral cancer, bronchial cancer, nasopharyngeal cancer, laryngeal cancer, skin cancer, hematologic cancer, parathyroid cancer, and ureteral cancer.

3. The marker composition of claim 1, wherein the immunotherapy is selected from the group consisting of NK cell therapy, T cell therapy, CAR-immune cell therapy, dendritic cell (DC) vaccine, anti-PD-L1, anti-PD-1, and anti-CTLA-4.

4. A composition for predicting immunotherapy responsiveness in a cancer patient, comprising an agent for measuring an mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G.

5. The composition of claim 4, wherein the expression level of the gene is measured in a region of interest within a tumor microenvironment of lesion tissue derived from a cancer patient.

6. The composition of claim 5, wherein the region of interest is a tumor region where PanCK is expressed, an immune region where CD45 is expressed, or a stromal region.

7. The composition of claim 4,
wherein the expression levels of NKG7, ULBP3, FPR2, or MYC are measured in the immune region where CD45 is expressed;
the expression levels of CXCL10 or NECTIN2 are measured in the tumor region where PanCK is expressed; and
the expression levels of CD8A, HLA-DQA1, BMP2, INF-β, TNF-β, IL6, OX40-L, OX40, Tim3, HLA-C, or HLA-G are measured in the stromal region.

8. The composition of claim 4, wherein the agent for measuring an mRNA expression level is a primer or probe that specifically binds to the gene.

9. The composition of claim 4, wherein the agent for measuring the protein expression level is an antibody that specifically binds to the protein encoded by the gene.

10. The composition of claim 4, wherein the cancer is selected from the group consisting of ovarian cancer, cholangiocarcinoma, colon cancer, adenocarcinoma, rectal cancer, breast cancer, synovial sarcoma, chondrosarcoma, thyroid cancer, gastric cancer, thymic cancer, cervical cancer, glioma, brain cancer, melanoma, lung cancer, bladder cancer, prostate cancer, leukemia, renal cancer, liver cancer, colorectal cancer, pancreatic cancer, lymphoma, uterine cancer, oral cancer, bronchial cancer, nasopharyngeal cancer, laryngeal cancer, skin cancer, hematologic cancer, parathyroid cancer, and ureteral cancer.

11. The composition of claim 4, wherein the immunotherapy is selected from the group consisting of NK cell therapy, T cell therapy, CAR-immune cell therapy, dendritic cell (DC) vaccine, anti-PD-L1, anti-PD-1, and anti-CTLA-4.

12. A kit for predicting immunotherapy responsiveness in a cancer patient, comprising the composition of claim 1 or 4.

13. An information providing method for predicting immunotherapy responsiveness in a cancer patient, the method comprising the steps of:
(1) measuring an mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G from a biological sample isolated from a cancer patient; and
(2) predicting responsiveness of the cancer patient to immunotherapy based on the measured mRNA or protein expression level.

14. The method of claim 13, wherein the expression level in step (1) is measured in a region of interest within a tumor microenvironment of the biological sample.

15. The method of claim 14, wherein the region of interest is a tumor region where PanCK is expressed, an immune region where CD45 is expressed, or a stromal region.

16. The method of claim 14,
wherein the expression levels of NKG7, ULBP3, FPR2, or MYC are measured in the immune region where CD45 is expressed;
the expression levels of CXCL10 or NECTIN2 are measured in the tumor region where PanCK is expressed; and
the expression levels of CD8A, HLA-DQA1, BMP2, INF-β, TNF-β, IL6, OX40-L, OX40, Tim3, HLA-C, or HLA-G are measured in the stromal region.

17. The method of claim 16, wherein the step of (2) predicting responsiveness of the cancer patient to immunotherapy is carried out by determining that the cancer patient is highly responsive to immunotherapy if the expression level of NKG7 or ULBP3 is equal to or greater than a defined expression threshold or if the expression level of FPR2 or MYC is equal to or less than the defined expression threshold.

18. The method of claim 16, wherein the step of (2) predicting responsiveness of the cancer patient to immunotherapy is carried out by determining that the cancer patient is highly responsive to immunotherapy if the expression level of CXCL10 or NECTIN2 is equal to or less than a defined expression threshold.

19. The method of claim 16, wherein the step of (2) predicting responsiveness of the cancer patient to immunotherapy is carried out by determining that the cancer patient is highly responsive to immunotherapy if the expression level of CD8A, HLA-DQA1, BMP2, INF-b, TNF-b, IL6, OX40-L, OX40, Tim3, HLA-C, or HLA-G is equal to or greater than a defined expression threshold.

20. An information providing method for survival prognosis of a cancer patient, the method comprising a step of measuring an mRNA or protein expression level of at least one gene selected from the group consisting of NKG7, ULBP3, FPR2, MYC, CXCL10, NECTIN2, CD8A, HLA-DQA1, BMP2, INF-β, TNF-β, IL6, OX40-L, OX40, Tim3, HLA-C, and HLA-G from a biological sample isolated from a cancer patient.

21. The method of claim 20, wherein the expression level is measured in a region of interest within a tumor microenvironment of the biological sample, the region being a tumor region where PanCK is expressed, an immune region where CD45 is expressed, or a stromal region.

22. The method of claim 21, wherein the cancer patient is determined to have high progression-free survival (PFS) if, in the immune region expressing CD45, the expression level of NKG7 or ULBP3 is equal to or greater than a defined expression threshold or the expression level of FPR2 or MYC is equal to or less than the defined threshold.

23. The method of claim 21, wherein the cancer patient is determined to have high progression-free survival (PFS) if the expression level of CXCL10 or NECTIN2 is equal to or less than a defined expression threshold in the tumor region where PanCK is expressed.

24. The method of claim 21, wherein the cancer patient is determined to have high progression-free survival (PFS) if the expression level of CD8A, HLA-DQA1, BMP2, INF-b, TNF-b, IL6, OX40-L, OX40, Tim3, HLA-C, or HLA-G is equal to or greater than a defined expression threshold in the stromal region.
